(19) Europäisches Patentamt
European Patent Office
Office européen des brevets

(11) **EP 4 431 906 A1**

(12) **EUROPEAN PATENT APPLICATION**
published in accordance with Art. 153(4) EPC

(43) Date of publication:
**18.09.2024 Bulletin 2024/38**

(21) Application number: **22892488.2**

(22) Date of filing: **13.10.2022**

(51) International Patent Classification (IPC):
**G01N 3/56** (2006.01)

(52) Cooperative Patent Classification (CPC):
**G01N 3/56**

(86) International application number:
**PCT/JP2022/038211**

(87) International publication number:
**WO 2023/084988 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.11.2021 JP 2021183404**

(71) Applicant: **SUMITOMO ELECTRIC INDUSTRIES, LTD.**
**Osaka-shi, Osaka 541-0041 (JP)**

(72) Inventors:
• **SUMIYA, Hitoshi**
**Osaka-shi, Osaka 541-0041 (JP)**
• **LEE, Jin Hwa**
**Osaka-shi, Osaka 541-0041 (JP)**

(74) Representative: **Boult Wade Tennant LLP**
**Salisbury Square House**
**8 Salisbury Square**
**London EC4Y 8AP (GB)**

(54) **CONTACT, AND METHOD FOR EVALUATING MICROWEAR PROPERTIES OF SINGLE-CRYSTAL DIAMOND USING SAME**

(57) A ring-shaped contact comprising a multi-crystal diamond formed from a plurality of diamond particles, said contact being formed in a manner such that the axis of rotation passes through the center thereof, and being equipped with a first section which includes an inside end section and has a constant thickness in the radial direction, and a second section which includes an outside end section and has a thickness which decreases in the radial direction, wherein: the second section has a first surface which is contiguous with the upper surface of the first section, a second surface which is contiguous with the bottom surface of the first section, and a connecting surface which connects the first surface and the second surface and includes the outside end section; the angle θ formed between a first line segment describing the first surface and a second line segment describing the second surface in a cross-section along the axis of rotation is 100-150°, inclusive; the length between a first boundary section, which is the boundary between the first surface and the connecting surface, and a second boundary section, which is the boundary between the second surface and the connecting surface, is 1-10μm, inclusive; the length from the axis of rotation to the outside end section is 0.5-5mm, inclusive; and the average particle diameter of the plurality of diamond particles is 10-300nm, inclusive.

FIG. 3

## Description

TECHNICAL FIELD

The present disclosure relates to a contactor and an evaluation method of a minute abrasion characteristics of monocrystalline diamond using the contactor. This application claims priority based on Japanese Patent Application No. 2021-183404 filed on November 10, 2021, and the entire contents of the Japanese patent application are incorporated herein by reference.

BACKGROUND ART

In industrial monocrystalline diamond, the nitrogen impurity and the distribution state of crystal defects differ from crystal to crystal, and even in the same crystal, the abrasion characteristics differ from region to region in the minute region. Therefore, particularly when using monocrystalline diamond as a tool material for precision machining, it is important to sufficiently understand the difference in abrasion characteristics between minute regions in the same crystal.

In order to evaluate the abrasion characteristics of a minute region of monocrystalline diamond, a method using a cast iron wheel or a metal bond diamond grinding wheel having a small diameter and a V-shaped cutting edge is known (Non-patent literature 1, Non-patent literature 2).

CITATION LIST

NON PATENT LITERATURE

Non-patent literature 1: Eileen M.Wilks&J.Wilks. (1959)."The Resistance of Diamond to Abrasion". Philosophical. Magazine, 4:38, 158-170.
Non-patent literature 2: E M Wilks and J Wilks. (1972). "The resistance of diamond to abrasion". Journal of Physics D: Applied Physics, 5, 1902 -1919.

SUMMARY OF THE INVENTION

A contactor of the present disclosure is a contactor having an annular shape and being made of polycrystalline diamond including a plurality of diamond particles, the contactor being configured to have a rotation axis extending through a center of the contactor. The contactor includes a first portion including an inner end portion, the first portion having a constant thickness in a radial direction, and a second portion including an outer end portion, the second portion having a decreasing thickness in the radial direction. The second portion has a first surface continuous with an upper surface of the first portion, a second surface continuous with a lower surface of the first portion, and a connection surface connecting the first surface and the second surface to each other and including the outer end portion. In a cross-section of the contactor along the rotation axis, an angle $\theta$ formed by a first line segment indicating the first surface and a second line segment indicating the second surface is 100° to 150°, a length between a first boundary portion and a second boundary portion is 1 $\mu$m to 10 $\mu$m, the first boundary portion being a boundary between the first surface and the connection surface, the second boundary portion being a boundary between the second surface and the connection surface, and a length from the rotation axis to the outer end portion is 0.5 mm to 5 mm. The plurality of diamond particles have an average particle diameter of 10 nm to 300 nm.

The evaluation method of the present disclosure is an evaluation method of evaluating the abrasion characteristics of the minute region of the monocrystalline diamond. The evaluation method includes the first step of forming an abrasion mark on the monocrystalline diamond by, while rotating the contactor, pressing the monocrystalline diamond against the outer end portion, and the second step of evaluating the abrasion characteristics of the minute region of the monocrystalline diamond based on a length of the abrasion mark.

BRIEF DESCRIPTION OF THE DRAWINGS

FIG. 1 is a photo substitutional view showing an example of the appearance of a contactor according to an embodiment of the present disclosure.
FIG. 2 is a top view of an example of a contactor in accordance with an embodiment of the present disclosure.
FIG. 3 is a XI-XI-line cross-sectional view of the contactor shown in FIG. 2.
FIG. 4 is an enlarged view of the second portion of the contactor shown in FIG. 3.
FIG. 5 is a cross-sectional view of a second portion in accordance with another embodiment of the present disclosure.
FIG. 6 is a schematic view of an abrasion testing apparatus used in the evaluation method of abrasion characteristics in a minute region of a monocrystalline diamond according to an embodiment of the present disclosure.
FIG. 7 is a diagram showing a <100> direction and a <110> direction of a (001) plane of the monocrystalline diamond.
FIG. 8 is a photo substitutional view showing the abrasion mark formed in Example 1.
FIG. 9 is a photo substitutional view showing the abrasion mark formed in Example 2.
FIG. 10 is a graph showing the relationship between the test number and the length of the abrasion mark in Example 1 and Example 2.

FIG. 11 is a photo substitutional view showing an ultraviolet-exited fluorescent image of a test piece A.

FIG. 12 is a graph showing the relationship between the test number and the length of the abrasion mark in the test piece A.

FIG. 13 is a photo substitutional view showing an ultraviolet-exited fluorescent image of a test piece B.

FIG. 14 is a graph showing the relationship between the test number and the length of the abrasion mark in the test piece B.

## DETAILED DESCRIPTION

[Problems to be Solved by the Present Disclosure]

The cast iron wheel described in Non-patent literature 1 is soft with respect to the monocrystalline diamond, and therefore, the cutting edge is likely to lose its shape during the abrasion test, and no abrasion mark is formed depending on the abrasion direction. Further, diamond powder of the abrasive is scattered, and the sharpness is quickly reduced. Therefore, it is difficult to quantitatively and appropriately evaluate the abrasion characteristics in the minute region of the monocrystalline diamond.

When the metal bond diamond grinding wheel described in patent literature 2 is used for a monocrystalline diamond material, diamond abrasive grains are likely to fall off, and the cutting edge is likely to lose its shape. In addition, due to the variation in the grain size and strength of the diamond abrasive grain, the grinding performance of the cutting edge is not stable. Therefore, it is difficult to quantitatively and appropriately evaluate the abrasion characteristics in the minute region of the monocrystalline diamond.

Therefore, an object of the present disclosure is to provide a contactor used in an evaluation method of abrasion characteristics in a minute region of a monocrystalline diamond and an evaluation method of abrasion characteristics in a minute region of a monocrystalline diamond using the same.

[Effects of Present Disclosure]

According to the present disclosure, it is possible to provide the contactor used for the abrasion characteristics in the minute region of the monocrystalline diamond. Further, it is possible to evaluate the abrasion characteristics in the minute region of the monocrystalline diamond by using the contactor.

[Description of Embodiments of Present Disclosure]

First, embodiments of the present disclosure will be listed and described.

    (1) A contactor of the present disclosure includes a contactor having an annular shape and being made of polycrystalline diamond including a plurality of diamond particles, the contactor being configured to have a rotation axis extending through a center of the contactor. The contactor includes a first portion including an inner end portion, the first portion having a constant thickness in a radial direction, and a second portion including an outer end portion, the second portion having a decreasing thickness in the radial direction. The second portion has a first surface continuous with an upper surface of the first portion, a second surface continuous with a lower surface of the first portion, and a connection surface connecting the first surface and the second surface to each other and including the outer end portion. In a cross-section of the contactor along the rotation axis, an angle θ formed by a first line segment indicating the first surface and a second line segment indicating the second surface is 100° to 150°, a length between a first boundary portion and a second boundary portion is 1 μm to 10 μm, the first boundary portion being a boundary between the first surface and the connection surface, the second boundary portion being a boundary between the second surface and the connection surface, and a length from the rotation axis to the outer end portion is 0.5 mm to 5 mm. The plurality of diamond particles have an average particle diameter of 10 nm to 300 nm.

According to the present disclosure, the abrasion characteristics in the minute region of the monocrystalline diamond (in the present specification, the abrasion characteristics in the minute region is also referred to as "minute abrasion characteristics") is described as follows.

(2) The polycrystalline diamond may have a Knoop hardness of 120 GPa or more. According to this, the accuracy of the evaluation of the minute abrasion characteristics is improved.

(3) A line of intersection of the connection surface and a cross-section including the rotation axis may be a straight line. According to this, the evaluation result of the minute abrasion characteristics is stabilized.

(4) A length from the rotation axis to an outer end portion may be larger than a length from the rotation axis to the first boundary portion and may be larger than a length from the rotation axis to the second boundary portion. According to this, the evaluation result of the minute abrasion characteristics is stabilized.

(5) The evaluation method of the present disclosure includes an evaluation method of evaluating abrasion characteristics of a minute region of a monocrystalline diamond. The evaluation method includes the first step of forming an abrasion mark on the monocrystalline diamond by, while rotating the contactor, pressing the monocrystalline diamond against the outer end portion, and the second step of evaluating the abrasion characteristics of the minute region of the monocrystalline diamond based on a length of the abrasion mark.

According to the present disclosure, it is possible to evaluate the abrasion characteristics in the minute region of the monocrystalline diamond.

(6) The monocrystalline diamond may have a plane. The first step of forming may include, a 1-1 step of arranging the monocrystalline diamond such that the plane faces the contactor and is parallel to the rotation axis, and a 1-2 step of pressing the monocrystalline diamond against the outer end portion by applying a load to the monocrystalline diamond in a normal direction to a third plane.

According to this, the accuracy of the evaluation of the minute abrasion characteristics is improved.

(7) The third plane may be a (001) plane. The abrasion mark may be parallel to a <100> direction of the (001) plane. According to this, the accuracy of the evaluation of the minute abrasion characteristics is improved.

[Details of Embodiments of Present Disclosure]

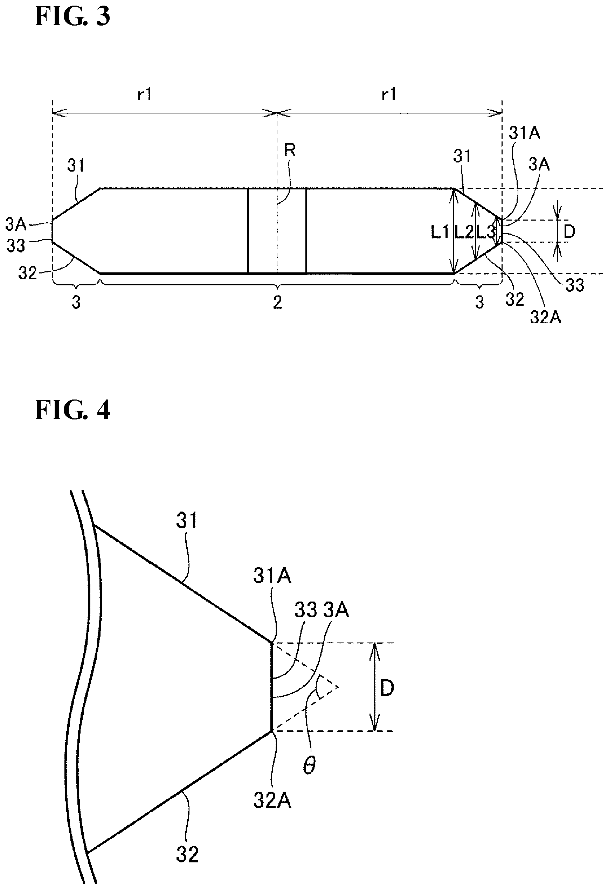

The contactor of the present disclosure and the evaluation method of the minute abrasion characteristics of monocrystalline diamond using the contactor will be described below with reference to the drawings. In the drawings of the present disclosure, the same reference numerals denote the same or corresponding parts. In addition, the dimensional relationships such as length, width, thickness, and depth are appropriately changed for the sake of clarity and simplification of the drawings, and do not necessarily represent the actual dimensional relationships. It is noted that, for convenience of explanation, FIG. 3, FIG. 4, and FIG. 5 are compressed in the vertical direction.

In the present description, the notation in the form of "A to B" means the upper limit and the lower limit of the range (that is, A or more and B or less), and in a case where a unit is not described in A and a unit is described only in B, the unit of A and the unit of B are the same.

[Embodiment 1: Contactor]

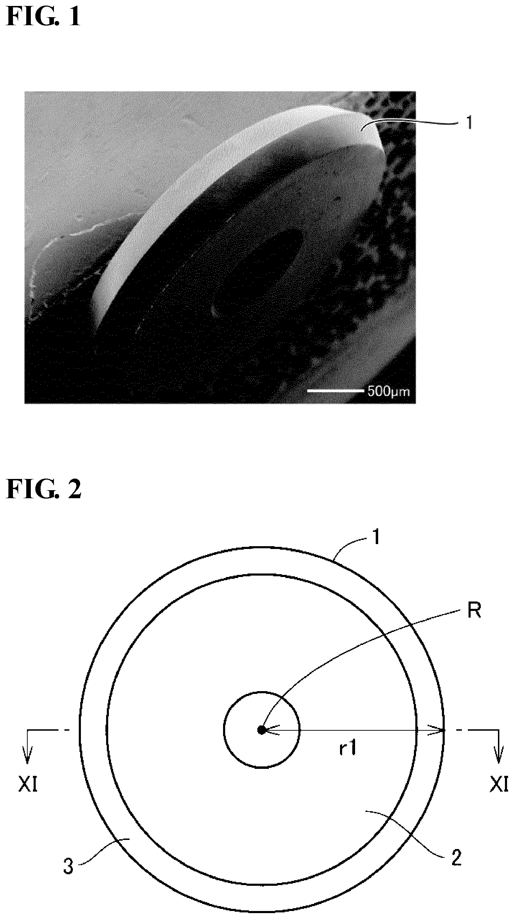

The contactor of an embodiment of the present disclosure (hereinafter also referred to as "the embodiment") will be described with reference to FIG. 1 to FIG. 5. A contactor 1 of the embodiment is a contactor having an annular shape and made of a polycrystalline diamond including a plurality of diamond particles, the contactor being configured to have a rotation axis R extending through a center of the contactor. Contactor 1 includes a first portion 2 having a constant thickness in a radial direction and a second portion 3 having a thickness decreasing in the radial direction. First portion 2 includes an inner end portion. Second portion 3 includes an outer end portion 3A. Second portion 3 has a first surface 31 continuous with an upper surface of first portion 2, second surface 32 continuous with a lower surface of first portion 2, and a connection surface 33 connecting first surface 31 and second surface 32 to each other and including outer end portion 3A. In a cross-section along rotation axis R, the angle θ formed by a first line segment indicating first surface 31 and a second line segment indicating second surface 32 is 100° to 150°, a length between a first boundary portion 31A and a second boundary portion 32A is 1 μm to 10 μm, first boundary portion 32A being a boundary between first surface 31 and connection surface 33, second boundary portion 32A being a boundary between second surface 32 and connection surface 33, and a length from rotation axis R to outer end portion 3A is 0.5 mm to 5 mm. An average particle diameter of the plurality of diamond particles is 10 nm to 300 nm.

The contactor of the present embodiment is made of a polycrystalline diamond including a plurality of diamond particles. Here, the polycrystalline diamond including a plurality of diamond particles means the polycrystalline diamond in which diamond particles are directly bonded to each other. The polycrystalline diamond is a polycrystalline substance made of a diamond single phase, which does not contain a bonding phase (binder) formed by one or both of a sintering aid and a binder generally used for a diamond sintered body.

The polycrystalline diamond may include inevitable impurities in addition to the diamond component as long as the effect of the present disclosure is exhibited. Examples of the inevitable impurities include hydrogen (H), oxygen (O), nitrogen (N), sodium (Na), magnesium (Mg), aluminum (Al), silicon (Si), phosphorus (P), sulfur (S), chlorine (Cl), potassium (K), calcium (Ca), titanium (Ti), iron (Fe), and molybdenum (Mo).

The content of the diamond component in the polycrystalline diamond is preferably 99 vol% or more. The polycrystalline diamond is made of the diamond component and inevitable impurities, and the content of the diamond component in the polycrystalline diamond is preferably 99 vol% or more. The fact that the polycrystalline diamond contains 99 vol% or more of the diamond component can be confirmed by an X-ray diffraction method. The fact that the polycrystalline diamond does not contain the bonding phase can be confirmed by observing the surface of the polycrystalline diamond with an optical microscope or an electron microscope.

The average particle diameter of a plurality of diamond particles included in the polycrystalline diamond (hereinafter, also referred to as "average particle diameter of diamond particles") is 10 nm to 300 nm. That is, the polycrystalline diamond is Nano Polycrystalline Diamond (NPD) in which fine diamond particles of several tens of nanometers are firmly bonded. The hardness of polycrystalline diamond has no orientation dependency, and polycrystalline diamond has higher hardness and strength than monocrystalline diamond.

The lower limit of the average particle diameter of the diamond particles may be 10 nm or more, may be 20 nm or more, or may be 30 nm or more, from the viewpoint of obtaining the mechanical strength peculiar to diamond. The upper limit of the average particle diameter of the diamond particles may be 300 nm or less, may be 200 nm or less, or may be 100 nm or less, from the viewpoint that a polycrystalline diamond 75 may exhibit isotropic hardness and abrasion resistance with respect to all orientations. The average particle diameter of the diamond particles may be 10 nm to 300 nm, may be 20 nm to 200 nm, or may be 30 nm to 100 nm.

The average particle diameter of the diamond particles is determined by a cutting method using a scanning electron microscope (SEM). Specifically, first, the polycrystalline diamond is observed at a magnification of 1,000 to 100,000 times using the scanning electron microscope to obtain an SEM image.

Next, a circle is drawn on the SEM image, and eight straight lines are drawn radially from the center of the circle (so that the crossing angle between each straight line is substantially equal) to the outer circumference of the circle. In this case, the observation magnification and the diameter of the circle are set such that the number of diamond particles (crystal particles) crossed by one straight line is about 10 to 50.

Next, the number of diamond particles crossing the crystal grain boundary is counted for each straight line, the length of the straight line is divided by the number of crossings to obtain the average intercept length, and the average intercept length is multiplied by 1.128 to obtain the average particle diameter. The above measurement is performed on three SEM images, and the average particle diameter is obtained for each of the three SEM images. The average value of the average particle diameters of the three SEM images is defined as the average particle diameter of the diamond particle in the present specification.

It was confirmed that, as far as the measure is concerned, the measurement results hardly vary even when the measurement was performed a plurality of times by changing the selected portion of the measurement visual field, and the measurement visual field does not become arbitrary even when the measurement visual field is set at random.

When the abrasion characteristics in the minute region of a monocrystalline diamond 75 are evaluated using contactor 1, outer end portion 3A of contactor 1 is brought into contact with monocrystalline diamond 75 while rotating contactor 1 around rotation axis R as a center, and an abrasion mark is formed on monocrystalline diamond 75. Therefore, the hardness of the polycrystalline diamond included in the contactor of the present embodiment may be equal to or greater than the hardness of monocrystalline diamond 75.

The hardness of monocrystalline diamond 75 varies from 70 to 120 GPa depending on the plane orientation. Therefore, the Knoop hardness of the polycrystalline diamond may be 120 GPa or more, 125 GPa or more, or 130 GPa or more. The upper limit of the Knoop hardness of the polycrystalline diamond is not particularly limited, but may be 160 GPa or less from the viewpoint of production. The Knoop hardness of the polycrystalline diamond may be 120 GPa to 160 GPa, may be 125 GPa to 155 GPa, or may be 130 GPa to 150 GPa.

In the present specification, the Knoop hardness of the polycrystalline diamond is measured under the conditions defined in JIS Z 2251:2009. In particular, a rhombic Knoop indenter is pressed into the polycrystalline diamond surface to apply a load for 10 seconds with a test force of 4.9 N, thereby forming an indentation. The test temperature is 23°C ± 5°C. After the test force is released, the length a ($\mu$m) of the longer diagonal line of the indentation remaining on the surface of the polycrystalline diamond is measured, and the Knoop hardness (HK) is calculated from the following Equation (1).

$$ HK = 14229 \times F/a^2 \quad \text{Equation (1)} $$

As shown in FIG. 1 to FIG. 3, contactor 1 includes first portion 2 and second portion 3 that is continuous with first portion 2 and surrounds the outer circumference of first portion 2, and in a cross-section including rotation axis R, the length of second portion 3 in rotation axis R direction decreases toward outer end portion 3A. Here, the length of rotation axis R direction of second portion 3 decreasing toward outer end portion 3A means that, as shown in FIG. 3, when the length of rotation axis R direction of second portion 3 is L1, L2, and L3 in order of closeness to the rotation axis, the relationship L1> L2> L3 is satisfied. As shown in FIG. 2, contactor 1 is a circle diameter in top view. The maximum length of contactor 1 in rotation axis R direction is not particularly limited, but can be set to, for example, 0.2 mm to 2 mm.

A length r1 in a direction orthogonal to rotation axis R between rotation axis R of contactor 1 and outer end portion 3A is 0.5 mm to 5 mm. According to this, contactor 1 can be easily handled, and the minute abrasion mark can be formed on monocrystalline diamond 75.

The lower limit of the length r1 may be 0.5 mm or more, may be 1 mm or more, or may be 1.5 mm or more, from the viewpoint of ease of handling of contactor 1. The upper limit of the length r1 may be 5 mm or less, may be 3 mm or less, or may be 2 mm or less, from the viewpoint of forming a minute abrasion mark. The length r1 may be 0.5 mm to 5 mm, may be 1 mm to 3 mm, or may be 1.5 mm to 2 mm.

The angle θ formed by first surface 31 and second surface 32 of contactor 1 is 100° to 150°, a length D in rotation axis R direction between first boundary portion 31A which is a boundary between first surface 31 and connection surface 33, and second boundary portion 32A which is a boundary between second surface 32 and connection surface 33 is 1 $\mu$m to 10 $\mu$m. In the present description, the angle θ formed by first surface 31 and second surface 32 means an angle formed by virtual planes obtained by enlarging first surface 31 and second surface 32 as indicated by a dotted line in FIG. 4. According to this, the abrasion mark formed on monocrystalline diamond 75 becomes clear, and a stable abrasion test can be performed. Therefore, the variation in the evaluation result of the minute abrasion characteristics is suppressed, and the evaluation result is stabilized. In the present description, the step of forming the abrasion mark on monocrystalline diamond 75 using contactor 1, which is performed for evaluation of the minute abrasion characteristics of monocrystalline diamond 75, is also referred to as an abrasion test.

The lower limit of the angle θ may be 100° or more, or may be 110° or more, from the viewpoint of maintaining the shape of outer end portion 3A during the abrasion test. The upper limit of the angle θ may be 150° or less, or may be 140° or less, from the viewpoint of forming a clear abrasion mark. The angle θ may be 100° to 150°, or may be 110° to 140°.

The lower limit of the length D may be 1 μm or more, or may be 2 μm or more, from the viewpoint of maintaining the shape of outer end portion 3A during the abrasion test. The upper limit of the length D may be 10 μm or less, or may be 8 μm or less, from the viewpoint of forming a clear abrasion mark. The length D may be 1 μm to 10 μm, or may be 2 μm to 8 μm.

As shown in FIG. 4, the line of intersection between connection surface 33 and the cross-section including rotation axis R may be a straight line. According to this, a stable abrasion test can be performed. Therefore, the variation in the evaluation result of the minute abrasion characteristics is suppressed, and the evaluation result is stabilized.

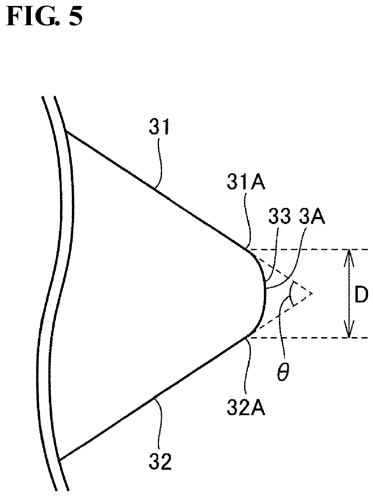

As shown in FIG. 5, the length from rotation axis R to outer end portion 3A may be larger than the length from rotation axis R to first boundary portion 31A and larger than the length from rotation axis R to second boundary portion 32A. According to this, a stable abrasion test can be performed. Therefore, the variation in the evaluation result of the minute abrasion characteristics is suppressed, and the evaluation result is stabilized. In the case where connection surface 33 is an arc in the cross-section including rotation axis R, the radius of the arc may be 1 μm to 10 μm, or may be 2 μm to 8 μm.

As shown in FIG. 1 to FIG. 3, first portion 2 of contactor 1 preferably includes a hole formed in a portion corresponding to rotation axis R. According to this, in the abrasion test, a spindle can be inserted into the hole. In addition, contactor 1 may include a shaft fixed to first portion 2.

An example of a method of manufacturing the contactor of the present embodiment will be described. First, high-purity graphite (purity of 99.9% or more) is used as a starting material and sintered by a direct conversion method under an ultrahigh pressure to synthesize a polycrystalline diamond. The sintering conditions may be, for example, a temperature of 2200 to 2300°C, a pressure of 15 to 16 GPa, and a sintering time of 10 to 30 minutes. The obtained polycrystalline diamond is formed into the shape of the contactor of the present embodiment by laser processing, grinding with a diamond grinding wheel, and polishing to obtain the contactor.

[Embodiment 2: Evaluation method of abrasion characteristics in minute region of monocrystalline diamond]

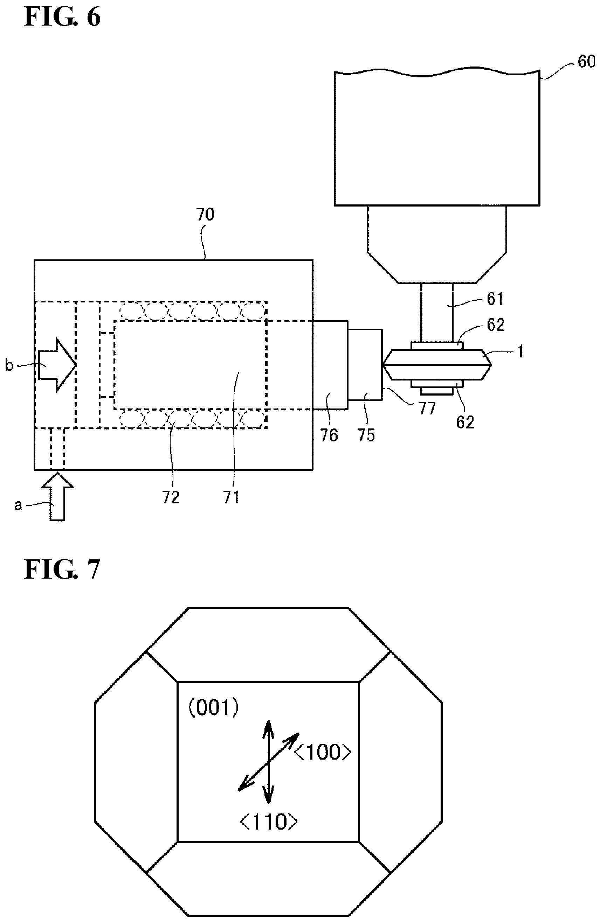

The evaluation method of the abrasion characteristics in the minute region of monocrystalline diamond 75 of the present embodiment will be described with reference to FIG. 6. FIG. 6 is a schematic view of an abrasion testing apparatus used in the evaluation method of the minute abrasion characteristics according to the present embodiment. The abrasion testing apparatus includes a machining center 60 and a sample holding portion 70. Machining center 60 includes a spindle 61 and a fixation screw 62 for fixing contactor 1 to spindle 61. Sample holding portion 70 includes a jig 76 for holding monocrystalline diamond 75, an air cylinder 71 for moving jig 76 in a contactor 1 direction, and a linear guide 72 disposed around air cylinder 71.

The evaluation method of the present disclosure is a method of abrasion characteristics of monocrystalline diamond in a minute region. The evaluation method incudes, a first step of forming an abrasion mark on monocrystalline diamond 75 by, while rotating contactor 1 according to the embodiment 1, pressing monocrystalline diamond 75 against outer end portion 3A, and a second step of evaluating the abrasion characteristics of monocrystalline diamond 75 in the minute region based on a length of the abrasion mark.

<First Step>

Spindle 61 of machining center 60 is inserted into a hole of contactor 1 and fixed by fixation screw 62. Since contactor 1 is fixed to spindle 61, when spindle 61 is rotated, contactor 1 is rotated in synchronization with the rotation of spindle 61. The rotational speed is preferably 100 rpm to 1000 rpm and/or the circumferential speed is preferably 1 m/min to 10 m/min from the viewpoint of suppressing thermal abrasion. In FIG. 6, contactor 1 is fixed to spindle 61 by fixation screw 62, but the method of fixing contactor 1 to spindle 61 is not limited to this. For example, contactor 1 and spindle 61 can be fixed to each other by using an adhesive.

Monocrystalline diamond 75 is fixed to jig 76 of sample holding portion 70. Air cylinder 71 is provided in the direction opposite to the direction in which monocrystalline diamond 75 is fixed to jig 76. As shown by the direction of arrow a in FIG. 6, by sending air of a constant pressure toward air cylinder 71, a load is applied to air cylinder 71 in the direction of arrow b, and air cylinder 71 moves in the direction of contactor 1. As a result, monocrystalline diamond 75 is pressed against outer end portion 3A of contactor 1, and the abrasion mark is formed in monocrystalline diamond 75. The pressing pressure is preferably 0.1 MPa to 0.2 MPa. The pressing time is preferably 60 seconds.

Monocrystalline diamond 75 may have a plane 77. The first step may include, a 1-1 step of disposing monocrystalline diamond 75 with plane 77 facing contactor 1 and being parallel to rotation axis R, and a 1-2 step of pressing monocrystalline diamond 75 against outer end portion 3A of contactor 1 by applying a load in a normal direction of plane 77 to monocrystalline diamond 75. This improves the accuracy of the minute abrasion test. When monocrystalline diamond 75 is processed to have plane 77, the (100) plane of monocrystalline diamond 75 is preferably parallel-polished with a metal bond diamond grinding wheel or a skeif.

Plane 77 of monocrystalline diamond 75 is the (001) plane, and the abrasion mark may be parallel to the <100> direction of the (001) plane. This improves the accuracy of the minute abrasion test.

When a plurality of abrasion marks are formed on one monocrystalline diamond 75, the plurality of abrasion marks are preferably substantially parallel to each other, and the abrasion mark interval can be 0.05 mm to 0.1 mm.

It is noted that, when first surface 31 and second surface 32 of contactor 1 are directly connected to form a V shape and connection surface 33 is not present before the start of the formation of the abrasion mark, the length D of outer end portion 3A is adjusted to be 1 μm to 10 μm by performing pretreatment five times or more under the above-described conditions (rotational speed, pressing pressure, and pressing time) for forming the abrasion mark in the <100> direction of the (001) plane of the monocrystalline diamond before the formation of the abrasion mark for evaluation.

< Second Step >

In the second step, the abrasion characteristics of monocrystalline diamond 75 is evaluated based on the length of the abrasion mark formed on monocrystalline diamond 75 in the first step. The abrasion characteristics can be evaluated by the abrasion amount (removal amount), the area of the abrasion mark, or the length of the abrasion mark. In the present embodiment, evaluation is performed by the length of the abrasion mark which can be easily measured. The length of the abrasion mark is measured with an optical microscope at an observation magnification of 500 times.

The evaluation method of the minute abrasion characteristics according to the present embodiment can evaluate the abrasion characteristics (abrasion resistance) of the minute region of monocrystalline diamond 75 with high accuracy. This enables the distribution state of the abrasion characteristics of monocrystalline diamond 75 due to the uneven distribution of impurities and crystal defects to be investigated in detail. This evaluation method is useful for selection and quality evaluation of monocrystalline diamond 75 when monocrystalline diamond 75 is used for precision cutting tools such as precision bit and small diameter end mill.

[Example]

The present embodiment will be described more specifically by examples. However, the present embodiment is not limited to these examples.

As test pieces, a high-purity, colorless transparent, synthetic monocrystalline diamond (type IIa, impurity: 0.1 ppm or less) containing almost no impurity or crystal defects and a yellow synthetic monocrystalline diamond (type Ib, containing about 100 ppm nitrogen as an isolated substitutional impurity) usually produced for industrial use were used. The (100) surface of each test piece was parallel polished with a metal bond diamond grinding wheel or a skeif plate to form a plane, and minute abrasion characteristics were evaluated on the plane.

It is presumed that the abrasion characteristics of the monocrystalline diamond are affected by impurities and crystal defects. Therefore, first, the validity of this evaluation method was confirmed and the wear state and stability of the contactor were investigated in a test piece made of the synthetic monocrystalline diamond (hereinafter, also referred to as "synthetic IIa type monocrystalline diamond ") which contains almost no impurity or crystal defects (Example 1 and Example 2 described below). Further, the difference in the minute abrasion characteristics of the synthetic IIa type monocrystalline diamond depending on the orientation was evaluated (Example 3 described below).

Next, a test piece made of synthetic monocrystalline diamond (hereinafter, also referred to as "synthetic Ib type monocrystalline diamond ") commonly produced for industrial use was used to investigate the relationship between growth sectors and the minute abrasion characteristics (Example 4 described below).

In all of the following examples, the contactor is made of polycrystalline diamond including a plurality of diamond particles. The average particle diameter of the plurality of diamond particles is 50 nm. Knoop hardness of the polycrystalline diamond is 130 GPa.

In all of the following examples, the conditions for forming the abrasion marks were a rotational speed 313 rpm (peripheral speed 3.15 m/min) of the contactor, a pressing pressure 0.1 MPa per one abrasion mark, and a pressing time 60 seconds. The interval between the abrasion marks on the test piece is 0.1 mm or 0.05 mm.

[Example 1: Evaluation of minute abrasion characteristics of (001) plane of synthetic IIa type monocrystalline diamond in <100> direction]

The contactor described in embodiment 1 was prepared as a contactor. The contactor has a cross-section shape as shown in FIG.3, the length r1 of 1.6 mm (in top view, a circle with a diameter of φ3.2 mm and the perimeter of the second portion is about 10 mm), the angle θ of 120°, and the maximum length (thickness) of 0.6 mm in rotation axis R direction, except that the outer end portion is V-shaped (that is, first surface 31 and second surface 32 intersect to form an angle θ, and connection surface 33 is not present).

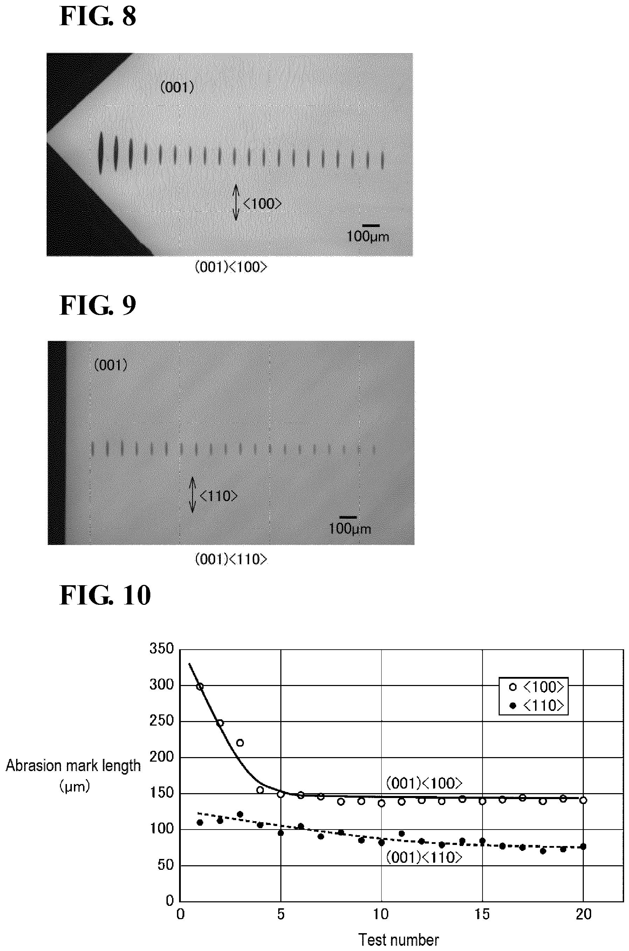

As shown in FIG.7 and FIG.8, the evaluation was performed by forming the abrasion mark in parallel in the <100> direction of the (001) plane with respect to the synthetic IIa type monocrystalline diamond. The result is shown as a solid line in the graph of FIG. 10. In the coordinate system of FIG. 10, the horizontal axis represents the test number, and the vertical axis represents the length (μm) of the abrasion mark. The test number corresponds to the number of times of formation of the abrasion mark. For example, test number 5 means the fifth formation of the abrasion mark.

As shown in FIG. 10, the length of the abrasion mark is gradually shortened until the fifth abrasion mark is formed as indicated by test number 5. This is presumably because the second portion of the contactor before the abrasion test is in a V-shaped sharp state, and thus abrasion marks are likely to be formed, and as the number of times of the pretreatment test increases, the tip of the second portion wears, and thus abrasion marks are unlikely to be formed. After the fifth abrasion mark is formed, the outer end portion is flat, and the length D of the outer end portion is about 5 to 6 μm. In the fifth and subsequent formation of the abrasion mark, the outer end portion is hardly worn and is stable, and thus the length and the width of the abrasion mark are substantially constant values. This is presumed to be because the abrasion resistance of the polycrystalline diamond is far superior to the abrasion resistance of the <100> direction of the (001) plane of monocrystalline diamond.

From the above, it was confirmed that by performing the formation of the abrasion mark five times or more as the pretreatment, the stable minute abrasion evaluation can be performed thereafter. In addition, it was confirmed that the stable abrasion test could be performed on the test piece made of the monocrystalline diamond.

[Example 2: Evaluation of Minute Abrasion Characteristics of (001) Plane of Synthetic IIa Monocrystalline Diamond in <110> Direction]

Then, as shown in FIG.7 and FIG.9, an abrasion mark was formed in parallel in the <110> direction of the (001) plane on the synthetic IIa type monocrystalline diamond using the same contactor as in Example 1, and evaluation was performed. The result is shown as a dotted line in the graph of FIG. 10.

As shown in FIG. 10, the abrasion mark is shortened as the test number (the number of times of formation of the abrasion mark) increases. This is presumably because the abrasion resistance of the polycrystalline diamond is at the same level as the abrasion resistance of the <110> direction of the (001) plane of the monocrystalline diamond, and thus the outer end portion of the contactor continues to wear little by little.

From the results of Example 1 and Example 2, it was confirmed that, for the purpose of examining the difference in the abrasion resistance between crystals and the distribution state of the abrasion characteristics in a crystal, it is preferable to perform evaluation by forming an abrasion mark parallel to the <100> direction of the (001) plane of the monocrystalline diamond.

[Example 3: Comparison of abrasion characteristics of <100> and <110> directions of (001) plane of synthetic IIa monocrystalline diamond]

In Example 1, the asymptotic value of the abrasion mark length (hereinafter, also referred to as "<100> abrasion mark length") of the (001) plane of synthetic IIa monocrystalline diamond in the <100> direction is about 145 to 150 μm. On the other hand, in Example 2, the abrasion mark length (hereinafter, also referred to as "<110> abrasion mark length") of the (001) plane of the synthetic IIa monocrystalline diamond in the <110> direction at the start of the test (test number 1) is about 110 to 120 μm. Therefore, the <100> abrasion mark length is about 1.3 times the <110> the abrasion mark length, and is about 2.2 times when converted into the abrasion volume.

The above results have the same tendency as the experimental results obtained by using the metal bond diamond grinding wheel of NPL 2, but the contactor of the present embodiment has very small wear (settling) of the outer end portion, and thus it is possible to more accurately evaluate the difference depending on the crystal or the difference depending on the place in the crystal.

It was confirmed from Example 1 to Example 3 that, according to the evaluation method using the contactor of the present embodiment, the difference in the abrasion characteristics (characteristic relating to the original abrasion resistance of diamond) of the monocrystalline diamond due to the plane orientation can be accurately evaluated even in the minute region of a level of several tens of micrometers.

[Example 4: Evaluation of relationship between the growth sector of synthetic Ib monocrystalline diamond and minute abrasion characteristic]

The contactor described in embodiment 1 was prepared as a contactor. The contactor has a cross-section shape shown in FIG. 3, and has the length r1 of 1.6 mm (in top view, a circle with a diameter of φ3.2 mm and the perimeter of the second portion is about 10 mm), the angle θ of 120°, the length D of 5 μm, and a maximum length (thickness) 0.6 mm in rotation axis R direction.

Using the contactor, the abrasion mark was formed in parallel in the <100> direction of the (001) plane on a test piece of synthetic Ib type monocrystalline diamond containing nitrogen impurities before and after 100 ppm, and the relationship between the grown sector and the minute abrasion characteristics was evaluated. Two test pieces were prepared. Hereinafter, they are referred to as the test piece A and the test piece B, respectively.

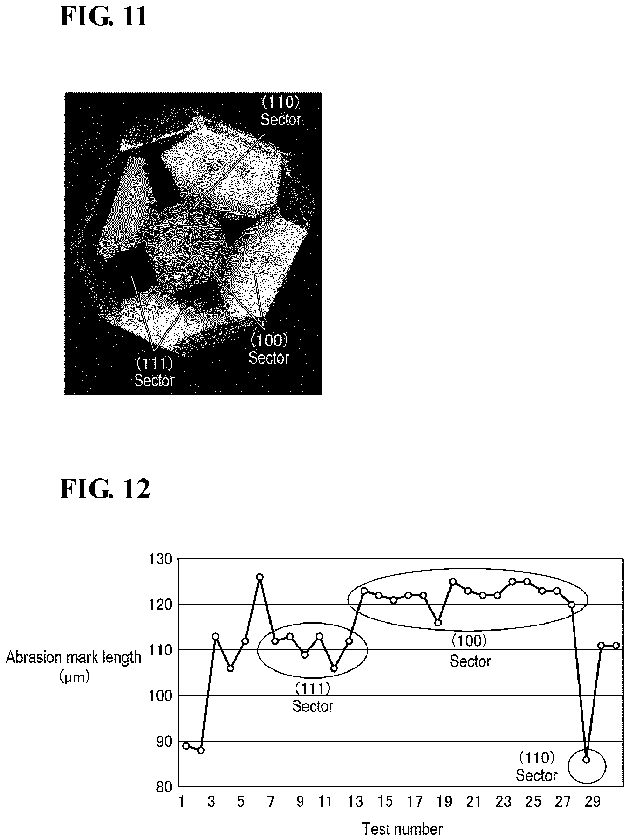

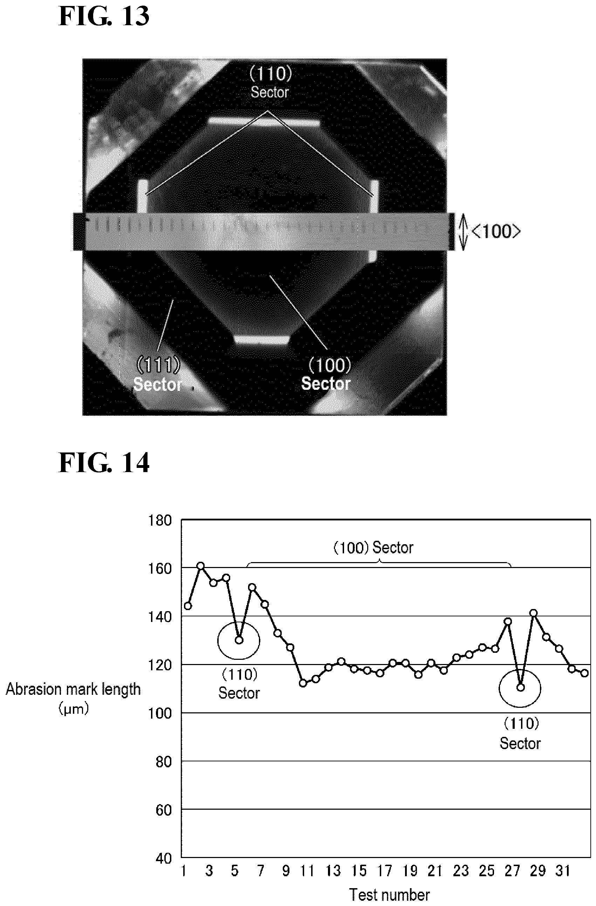

The distribution of the growth sectors in the test piece A and the test piece B is confirmed by ultraviolet-exited fluorescent image observation. FIG. 11 is a photo substitutional view showing an ultraviolet-exited fluorescent image of the test piece A. FIG. 13 is a photo substitutional view showing an ultraviolet-exited fluorescent image of the test piece B. In FIG. 13, the abrasion mark observed by the optical microscope is superimposed on the ultraviolet-exited fluorescent image.

FIG. 12 is a graph showing the relationship between the test number and the length of the abrasion mark in the test piece A. In FIG. 12, test number 7 to test number 12 indicate the abrasion marks formed in the (111) sector, test number 13 to test number 27 indicate the abrasion marks formed in the (100) sector, and test number 28 indicates the abrasion marks formed in the (110) sector.

FIG. 14 is a graph showing the relationship between the test number and the length of the abrasion mark in the test piece B. In FIG. 14, test number 5 indicates an abrasion mark formed in the (110) sector, test number 6 to test number 26 indicate abrasion marks formed in the (100) sector, and test number 27 indicates an abrasion mark formed in the (110) sector.

From FIG. 12 and FIG. 14, it was confirmed that the abrasion characteristics were different depending on the growth sector, and in particular, the abrasion resistance of the (110) sector was very high. It is known that the amount of nitrogen in each growth sector is typically (111)>(100)>(113)>(110), and the (110) sector contains very little nitrogen. Therefore, it is presumed that the abrasion amount of the (110) sector portion is small.

From the above, it was confirmed that the abrasion resistance of the monocrystalline diamond containing impurities was different depending on the growth sector and was not a little affected by the distribution state of the impurities by the evaluation method of the minute abrasion characteristics of the monocrystalline diamond using the contactor of the present embodiment.

Although the embodiments and examples of the present disclosure have been described above, it is originally intended that the configurations of the above-described embodiments and examples may be appropriately combined or variously modified. The embodiments and examples disclosed herein are illustrative in all respects and should not be construed as limiting. The scope of the present disclosure is defined by the appended claims rather than the foregoing embodiments and examples, and is intended to include all modifications within the scope and meaning equivalent to the appended claims.

REFERENCE SIGNS LIST

1 contactor
2 first portion
3 second portion
3A outer end portion
31 first surface
32 second surface
31A first boundary portion
32A second boundary portion
33 connection surface
R rotation axis
60 machining center
61 spindle
62 fixation screw
70 sample holding portion
71 air cylinder
72 linear guide
75 monocrystalline diamond
76 jig
77 plane

**Claims**

1. A contactor having an annular shape and being made of polycrystalline diamond including a plurality of diamond particles,
the contactor being configured to have a rotation axis extending through a center of the contactor, the contactor comprising:

   a first portion including an inner end portion, the first portion having a constant thickness in a radial direction; and
   a second portion including an outer end portion, the second portion having a decreasing thickness in the radial direction,
   wherein the second portion has
   a first surface continuous with an upper surface of the first portion, a second surface continuous with a lower surface of the first portion, and a connection surface connecting the first surface and the second surface to each other and including the outer end portion,
   wherein in a cross-section of the contactor along the rotation axis,
   an angle θ formed by a first line segment indicating the first surface and a second line segment indicating the second surface is 100° to 150°,
   a length between a first boundary portion and a second boundary portion is 1 μm to 10 μm, the first boundary portion being a boundary between the first surface and the connection surface, the second boundary portion being a boundary between the second surface and the connection surface, and

a length from the rotation axis to the outer end portion is 0.5 mm to 5 mm, and

wherein the plurality of diamond particles have an average particle diam eter of 10 nm to 300 nm.

**2.** The contactor according to claim 1, wherein the polycrystalline diamond has a Knoop hardness of 120 GPa or more.

**3.** The contactor according to claim 1 or 2, wherein a line of intersection of the connection surface and a cross-section including the rotation axis is a straigh t line.

**4.** The contactor according to claim 1 or 2, wherein a length from the rota tion axis to an end portion of the contactor is larger than a length from the rotatio n axis to the first boundary portion and is larger than a length from the rotation a xis to the second boundary portion.

**5.** An evaluation method of evaluating abrasion characteristics of a minute r egion of a monocrystalline diamond, the evaluation method comprising:

a first step of forming an abrasion mark on the monocrystalline diamond by, while rotating the contactor according to any one of claims 1 to 4, pressing t he monocrystalline diamond against the outer end portion; and

a second step of evaluating the abrasion characteristics of the minute reg ion of the monocrystalline diamond based on a length of the abrasion mark.

**6.** The evaluation method according to claim 5, wherein the monocrystalline diamond has a plane, and wherein the first step includes:

a 1-1 step of arranging the monocrystalline diamond such that the plane faces the contactor and is parallel to the rotation axis; and

a 1-2 step of pressing the monocrystalline diamond against th e outer end portion by applying a load to the monocrystalline diamond in a normal direction to the plane.

**7.** The evaluation method according to claim 6, wherein the plane of the m onocrystalline diamond is a (001) plane, and wherein the abrasion mark is parallel to a <100> direction of the (001) plane.

FIG. 1

FIG. 2

## FIG. 3

## FIG. 4

# FIG. 5

**FIG. 6**

**FIG. 7**

## FIG. 8

(001)<100>

## FIG. 9

(001)<110>

## FIG. 10

# FIG. 11

# FIG. 12

## FIG. 13

## FIG. 14

## INTERNATIONAL SEARCH REPORT

| International application No. |
| --- |
| **PCT/JP2022/038211** |

**A. CLASSIFICATION OF SUBJECT MATTER**

*G01N 3/56*(2006.01)i
FI:   G01N3/56 H

According to International Patent Classification (IPC) or to both national classification and IPC

**B. FIELDS SEARCHED**

Minimum documentation searched (classification system followed by classification symbols)

G01N3/00-3/62; C01B32/25

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | 李真和,真鍋佳典,寺本三記,原野佳津子,小林豊,角谷均. "各種ダイヤモンドの摩耗特性". ２０１９年度精密工学会春季大会学術講演会講演論文集. 13 March 2019, pp. 646, 647, Internet: <URL: https://doi.org/10.11522/pscjspe.2019S.0_646>, (LEE, Masakazu, MANABE, Keisuke, TERAMOTO, Minori, HARANO, Katsuko, KOBAYASHI, Yutaka, SUMIYA, Hitoshi. Wear characteristics of various diamonds), non-official translation (Proceedings of the 2019 JSPE Spring Meeting.) entire text, all drawings | 1-7 |
| A | WO 2019/069888 A1 (KUSANO, Eiji) 11 April 2019 (2019-04-11) entire text, all drawings | 1-7 |
| A | JP 2014-91661 A (SUMITOMO ELECTRIC IND. LTD.) 19 May 2014 (2014-05-19) entire text, all drawings | 1–7 |
| A | US 2011/0146372 A1 (VAREL EUROPE S.A.S.) 23 June 2011 (2011-06-23) entire text, all drawings | 1–7 |
| A | CN 105158098 A (NANJING UNIVERSITY OF AERONAUTICS AND ASTRONAUTICS) 16 December 2015 (2015-12-16) entire text, all drawings | 1–7 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **15 November 2022** | **29 November 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

# EP 4 431 906 A1

## INTERNATIONAL SEARCH REPORT
### Information on patent family members

International application No.

**PCT/JP2022/038211**

| Patent document cited in search report | | | Publication date (day/month/year) | Patent family member(s) | | | Publication date (day/month/year) |
|---|---|---|---|---|---|---|---|
| WO | 2019/069888 | A1 | 11 April 2019 | JP | 2019-66366 | A | |
| JP | 2014-91661 | A | 19 May 2014 | (Family: none) | | | |
| US | 2011/0146372 | A1 | 23 June 2011 | US | 2013/0239652 | A1 | |
| | | | | US | 2011/0146373 | A1 | |
| | | | | US | 2011/0146374 | A1 | |
| | | | | US | 2011/0148021 | A1 | |
| | | | | WO | 2011/075432 | A2 | |
| | | | | WO | 2011/075435 | A1 | |
| | | | | WO | 2011/075438 | A1 | |
| | | | | WO | 2011/075479 | A1 | |
| CN | 105158098 | A | 16 December 2015 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021183404 A

**Non-patent literature cited in the description**

- **EILEEN M.WILKS ; J.WILKS.** The Resistance of Diamond to Abrasion. *Philosophical. Magazine,* 1959, vol. 4 (38), 158-170

- **E M WILKS ; J WILKS.** The resistance of diamond to abrasion. *Journal of Physics D: Applied Physics,* 1972, vol. 5, 1902-1919